(19) Europäisches Patentamt
European Patent Office
Office européen des brevets

(11) Veröffentlichungsnummer: **0 272 668 B1**

(12) # EUROPÄISCHE PATENTSCHRIFT

(45) Veröffentlichungstag der Patentschrift: **10.06.92**

(51) Int. Cl.5: **C07D 493/04**, C07D 313/00, C12P 17/16, C12P 1/06, A61K 31/365, A01N 63/02, //(C07D493/04,311:00,313:00), (C12P1/06,C12R1:465)

(21) Anmeldenummer: **87118929.6**

(22) Anmeldetag: **21.12.87**

Die Akte enthält technische Angaben, die nach dem Eingang der Anmeldung eingereicht wurden und die nicht in dieser Patentschrift enthalten sind.

(54) **Amycin und dessen Derivate, Verfahren zu deren Herstellung und deren Verwendung.**

(30) Priorität: **24.12.86 DE 3644374**
**08.01.87 DE 3700325**

(43) Veröffentlichungstag der Anmeldung:
**29.06.88 Patentblatt 88/26**

(45) Bekanntmachung des Hinweises auf die Patenterteilung:
**10.06.92 Patentblatt 92/24**

(84) Benannte Vertragsstaaten:
**AT BE CH DE ES FR GB GR IT LI LU NL SE**

(56) Entgegenhaltungen:
**EP-A-01 789 09**
**GB-A- 1 254 721**

(73) Patentinhaber: **HOECHST AKTIENGESELLSCHAFT**
**Postfach 80 03 20**
**W-6230 Frankfurt am Main 80(DE)**

(72) Erfinder: **Hammann, Peter, Dr.**
**Mörikestrasse 6**
**W-6233 Kelkheim (Taunus)(DE)**
Erfinder: **Grabley, Susanne, Dr.**
**Hölderlinstrasse 7**
**W-6240 Königstein/Taunus(DE)**
Erfinder: **Voelskow, Hartmut, Dr.**
**Akazienstrasse 22**
**W-6234 Hattersheim am Main(DE)**
Erfinder: **Sachse, Burkhard, Dr.**
**An der Ziegelei 30**
**W-6233 Kelkheim (Taunus)(DE)**
Erfinder: **Raether, Wolfgang, Dr.**
**Falkensteinstrasse 6**
**W-6072 Dreieich(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**CHEMICAL ABSTRACTS, Band 101, Nr. 21, 19. November 1984, Columbus, Ohio, USA; GASSMANN P. et al.: "Metabolites of microorganisms. 226. Structural elucidation of niphimycin, part 3. Identity of scopafungin with niphimycin I and the position of the malonyl residue of niphimycin and copiamycin", Seite 726, Spalte 1, Zusammenfassung-Nr. 191 458s**

**CHEMICAL ABSTRACTS, Band 79, Nr. 5, 6. August 1973, Columbus, Ohio, USA; AGAINA S. et al.: "Chemistry of the antifungal antibiotic niphimycin", Seite 171, Spalte 2, Zusammenfassung-Nr. 28 662x**

Erfinder: **Giani, Carlo, Dr.**
**Hedderichstrasse 69**
**W-6000 Frankfurt am Main 70(DE)**
Erfinder: **Seibert, Gerhard, Prof. Dr.**
**Gläserweg 21**
**W-6100 Darmstadt(DE)**

## Beschreibung

Die Britische Patentschrift 1 254 721 beschreibt die Herstellung des Makrolid-Antibiotikums Niphimycin durch Fermentation von Streptomyces hygroscopicus. Niphimycin kann insbesondere als antifungisches Agens verwendet werden.

Die Europäische Patentschrift 178 909 beschreibt die Herstellung von Scopafungin bzw. Methylscopafungin, sowie von Methylamycin B.

Es wurde nun gefunden, daß der Stamm Streptomyces parvullus, der bei der Deutschen Sammlung von Mikroorganismen unter der Nummer DSM 3816 hinterlegt wurde, ein neues Antibiotikum, das Amycin, synthetisiert. Amycin kann als pharmazeutisch aktive Substanz breite Anwendung finden. Obwohl Amycin dem Niphimycin in der Strukturformel ähnlich ist, wurde überraschend festgestellt, daß es insbesondere eine signifikant stärkere antifungische Wirkung besitzt. Derivate des Amycins lassen sich ebenfalls als pharmazeutisch aktive Substanzen einsetzen.

Die Erfindung betrifft somit:

1. Verbindungen der allgemeinen Formel Ia

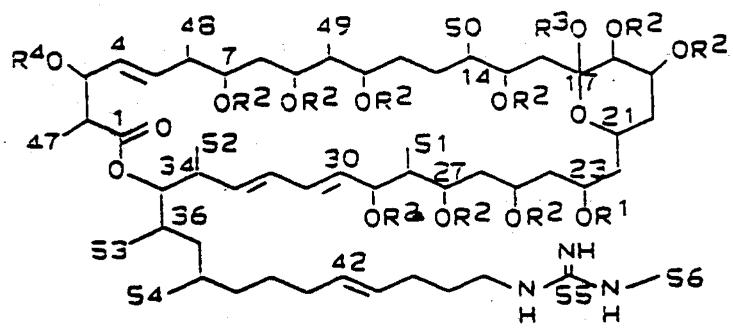

Ia

in der jeweils unabhängig voneinander

$R^1$ und $R^2$ Wasserstoff oder eine Malonylgruppe ist, wobei höchstens ein $R^2$-Substituent eine Malonylgruppe darstellt,

$R^3$ und $R^4$ Wasserstoff oder $C_1$-$C_6$ Alkyl oder Allyl bedeutet,

sowie die Verbindungen der Formel IIa, die in 22/23 Stellung ungesättigt sind,

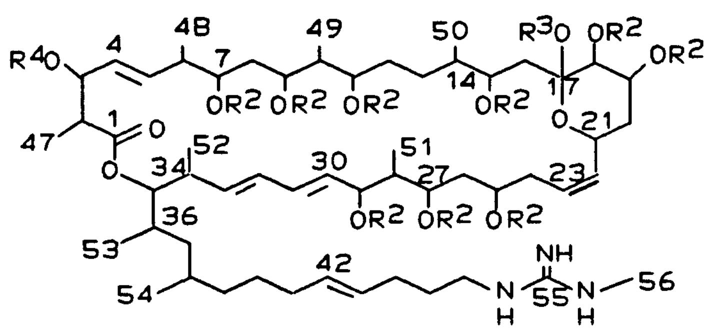

IIa

ausgenommen die Kombinationen in Verbindungen der Formel Ia, in denen

a) $R^1$ Malonyl und alle $R^2$, $R^3$, $R^4$ Wasserstoff,

b) $R^1$ Malonyl, alle $R^2$ und $R^4$ Wasserstoff und $R^3$ Methyl oder

c) $R^1$, alle $R^2$ und $R^4$ Wasserstoff und $R^3$ Methyl ist,

eingeschlossen weiterhin Verbindungen der Formel Ib,

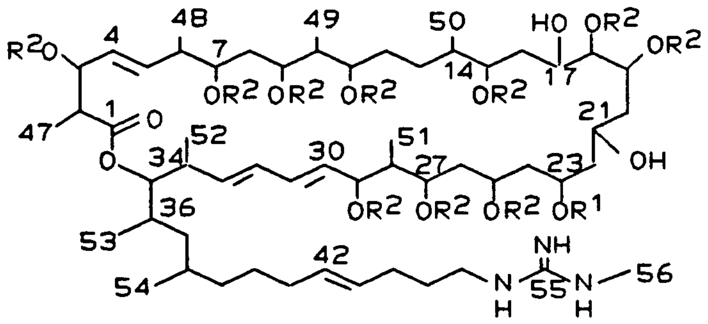

Ib

in der $R^1$ und $R^2$ die obengenannte Bedeutung haben, sowie die Verbindungen der Formel IIb, die in 22/23 Stellung ungesättigt sind,

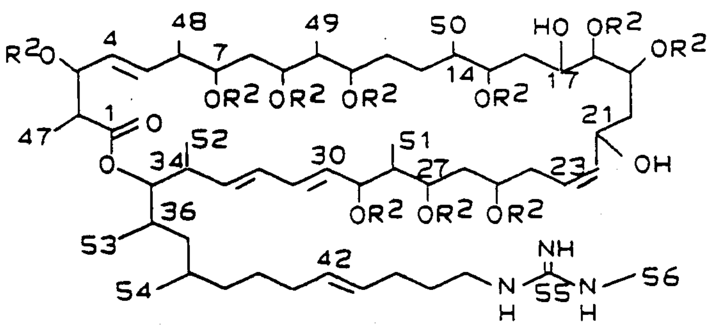

IIb

ausgenommen jene Verbindungen der Formel Ib, in welcher $R^1$ und alle $R^2$ Wasserstoff bedeuten.

2. Ein Verfahren zur Herstellung der Verbindungen der allgemeinen Formeln Ia, IIa und Ib und IIb, das dadurch gekennzeichnet ist, daß

a) Streptomyces parvullus, DSM 3816, sowie seine Varianten und Mutanten in einem Nährmedium kultiviert werden bis sich die Verbindung der Formel Ia, in der $R^1$ und ein $R^2$-Rest Malonyl sowie $R^3$ und $R^4$ Wasserstoff bedeutet, sowie die Verbindung, in der $R^1$, alle $R^2$, $R^3$ und $R^4$ Wasserstoff bedeutet, in der Kultur anhäuft

und diese gegebenenfalls

b) isoliert und derivatisiert wird.

3. Die Verwendung der Verbindungen der allgemeinen Formeln Ia, IIa und Ib und IIb als antimikrobielles Agens.

Im folgenden wird die Erfindung detailliert, insbesondere in ihren bevorzugten Ausführungsformen, beschrieben. Ferner wird die Erfindung in den Patentansprüchen definiert.

In einer Nährlösung, die eine Kohlenstoffquelle und eine Stickstoffquelle sowie die üblichen anorganischen Salz enthält, produziert Streptomyces parvullus, DSM 3816, das antimikrobiell wirksame Amycin, in dem $R^1$ und ein $R^2$-Rest Malonyl sowie $R^3$ und $R^4$ Wasserstoff bedeuten bzw. die Desmalonylverbindung, in der $R^1$, alle $R^2$, $R^3$ und $R^4$ Wasserstoff ist. Die $R^2$-Malonylgruppe im Amycin kann an eine der möglichen freien Hydroxylgruppen im Molekül gebunden sein. Anstelle des Stammes DSM 3816 können natürlich auch seine Mutanten und Varianten eingesetzt werden, soweit sie mindestens eine dieser Verbindungen synthetisieren Solche Mutanten können in an sich bekannter Weise durch physikalische Mittel, beispielsweise Bestrahlung, wie mit Ultraviolett- oder Röntgenstrahlen, oder chemische Mutagene, wie beispielsweise Ethansulfonsäuremethylester (Ethylmethylsulfononat, EMS) oder 2-Hydroxy-4-methoxybenzophenon (MOB), erzeugt werden.

Als bevorzugte Kohlenstoffquellen für die aerobe Fermentation eignen sich assimilierbare Kohlenhydrate und Zuckeralkohole, wie Glukose, Laktose oder D-Mannit sowie kohlenhydrathaltige Naturprodukte, wie Malzextrakt. Als stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. An anderen anorganischen Salzen kann die Nährlösung beispielsweise Chloride, Carbonate, Sulfate oder Phosphate der alkali- oder Erdalkalimetalle, Eisen, Zink und Mangan enthalten.

Die Bildung des Amycin verläuft besonders gut in einer Nährlösung, die Sojamehl und Mannit, insbesondere jeweils 2 % bezogen auf das Gewicht der gesamten Nährlösung, enthält. Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 35 ° C, vorzugsweise bei etwa 25 bis 30 ° C, insbesondere bei 28 bis 30 ° C erfolgen. Der pH-Bereich sollte zwischen 6 und 8 liegen, vorteilhaft zwischen 6,5 und 7,5. Unter diesen Bedingungen zeigt die Kulturbrühe im allgemeinen nach 1 bis 5 Tagen eine nennenswerte antimikrobielle Wirkung.

Vorteilhaft kultiviert man in mehreren Stufen, d.h. man stellt zunächst eine oder mehrere Vorkulturen in einem flüssigen Nährmedium her, die dann in das eingentliche Produktionsmedium, die Hauptkultur, beispielsweise im Volumenverhältnis 1:10, überimpft werden. Die Vorkultur erhält man Z.B., indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur oder dem Mycelvolumen, durch Dünnschichtchromatographie oder Ausprüfen der biologischen Aktivität überwacht werden. Amycin und die entsprechende Desmalonylverbindung sind sowohl im Mycel als auch im Kulturfiltrat enthalten.

Die Isolierung der genannten Verbindungen aus dem Kulturmedium erfolgt nach bekannten Methoden unter Berücksichtigung der chemische, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise auf Kieselgel mit n-Butanol/Essigsäure/Wasser als Laufmittel verwendet werden, wobei die Menge der gebildeten antimikrobiellen Substanz zweckmäßig mit einer Eichlösung verglichen wird.

Zur Isolierung der Verbindungen werden Kultrubrühe und Mycel zuerst mit organischen Lösungsmitteln, wie z.B. Chloroform, Essigester usw. extrahiert, um die unpolaren Verunreinigungen zu entfernen. Anschließend wird mit einem polareren Lösungsmittel, beispielsweise niederen Alkanolen oder Gemischen aus Chloroform und Essigester mit einem niederen Alkanol, extrahiert.

Die Reinisolierung erfolgt vorzugsweise an geeigneten Medien, wie z.B. Kieselgel, Aluminiumoxid oder Ionenaustauschern, durch anschließende Elution mit organischen, polaren Lösungsmitteln oder Lösungsmittelgemischen, wie z.B. Essigester, Gemischen aus Essigester und einem niederen Alkanol gegebenenfalls mit Wasser, bzw. einem für Ionenaustauscher geeigneten Salzgradienten, wie beispielsweise Kochsalz oder Tris-HCl, und Sammeln der Antibiotisch aktiven Fraktionen.

Reines Amycin ist amorph. Die Verbindung ist in polaren Lösungsmitteln, wie z.B. Wasser, Methanol und DMF löslich, mäßig löslich beispielsweise in höheren Alkoholen und unlöslich in unpolaren Lösungsmitteln, wie z.B. Chloroform, Essigester oder Ether.

Die Überführung in die Derivate des Amycins erfolgt in an sich bekannter Weise. So kann man die $R^3$-Alkyl/Allylverbindungen durch Behandlung von Amycin mit den entsprechenden Alkoholen unter sauren Bedingungen in organischen Lösungsmitteln herstellen. Geeignete Alkohole sind beispieslweise $C_1$-$C_6$ Alkanole oder Allylalkohol. Höherkettige Alkanole können ebenfalls verwendet werden, jedoch wird die Ausbeute der gewünschten Verbindung mit zunehmender Kettenlänge geringer. Bevorzugt setzt man den Alkohol selbst als Lösungsmittel ein. Die Reaktion kann bei -20 bis 40 ° C, bevorzugt jedoch bei Raumtemperatur stattfinden. Die Reaktionszeit ist abhängig vom Alkohol und dauert ungefähr 10 Minuten bis 4 Stunden.

Eine Abspaltung der Malonsäurehalbester im Amycin unter basischen, sauren oder neutralen Bedingungen in Wasser oder niederen Alkoholen, wie beispielsweise Methanol, Ethanol, Isopropanol usw. ergibt die Desmalonylverbindung. Als weitere Komponente erhält man die in 22/23 Stellung dehydratisierte Verbindung (Formel IIa). Die Reaktionstemperatur kann hier -30 bis 40 ° C betragen. Bevorzugt arbeitet man jedoch auch bei Raumtemperatur.

Die Derivatisierung der Desmalonylverbindung mit den entsprechenden Alkoholen unter sauren Bedingungen führt zu den $R^3$ Alkyl/Allyl-Acetalen. Für die Eignung der Alkohole gilt das gleiche wie oben im Zusammenhang mit der Herstellung der $R^3$-Alkylverbindungen schon aufgeführt. Bei dieser Reaktion entsteht auch das $R^3$/$R^4$-Dialkylderivat.

Das Halbacetal kann reduktiv beispielsweise mit Borhydriden und Aluminiumhydriden, wie $NaBH_4$ oder Lithiumaluminiumhydrid in polaren Lösungsmitteln, wie Wasser oder niederen Alkoholen etc., geöffnet werden.

Auch die Derivate des Amycins zeigen antimikrobielle Wirkung, insbesondere gegen bakterielle Keime, sowie humanpathogene, phytopathogene und lebensmittelverderbende Pilze. Amycin und seine Derivate zeichnen sich bei besserer Wirkung im Vergleich zu Niphimycin durch verminderte Toxizität und bessere Wasserlöslichkeit aus. Die Verbindungen sind im festen Zustand und in Lösung im pH-Bereich 3 bis 9,

insbesondere 5 bis 8, stabil und lassen sich damit in übliche galenische Zubereitungen einarbeiten.

In den sich anschließenden Beispielen wird die Erfindung weiter erläutert. Prozentangaben beziehen sich auf das Gewicht und Mischungsverhältnisse bei Flüssigkeiten beziehen sich auf das Volumen, wenn keine anderen Angaben gemacht wurden.

Beispiele

1.

a) Herstellung einer Sporensuspension von Streptomyces parvullus DSM 3816

Zusammensetzung des Nährmediums:
12,5 g Glycerin
1,0 g Arginin
1,0 g NaCl
1,0 g $K_2HPO_4$
0,5 g $MgSO_4.7H_2O$
15,0 g Agar
2,5 ml Spurenelementlösung
1 l $H_2O$ dest.

Zusammensetzung der Spurenelementlösung
30 g $CaCl_2.2H_2O$
1,0 g Eisen-III-citrat
0,2 g $M_nSO_4$
0,1 g $ZnCl_2$
0,025 g $CuSO_4.5H_2O$
0,02 g $Na_2B_4O_7.10H_2O$
0,004 g $CaCl_2$
0,01 g $Na_2MoO_4.2H_2O$
1 l $H_2O$ dest.

Schrägröhrchen mit obengenanntem Nährmedium werden mit DSM 3816 beimpft und 8 Tage bei 30° C bebrütet. Die Sporen der Röhrchen werden mit 3 ml einer Lösung aus 0,9 % NaCl und 0,1 % Tween 80 abgeschwemmt und bis zur Beimpfung bei 4° C aufbewahrt.

b) Herstellung einer Vorkultur von DSM 3816 im Erlenmeyerkolben

5 Erlenmeyerkolben von 300 ml Fassungsvermögen werden mit je 100 ml Nährlösung (40 g Glucose, 30 g Sojamehl, 2,5 NaCl, 2,5 g $CaCO_3$, in 1 l dest. Wasser, pH 7,5 vor der Sterilisation) beschickt und mit je 1,5 ml der nach 1a) hergestellten, möglichst frischen Sporensuspension angeimpft. Es wird auf einer Schüttelmaschine (180 UPM) bei 30° C 72 h inkubiert.

c) Herstellung einer Hauptkultur von DSM 3816

Ein 300 ml Erlenmeyerkolben mit 100 ml Nährlösung (20 g Sojamehl, 20 g Mannit, in 1 l dest. Wasser, pH 7,5 vor der Sterilisation) wird mit 3 ml der Vorkultur gemäß 1b beimpft und bei 30° C auf der Schüttelmaschine (180 UPM) inkubiert. Das Produktionsmaximum wird nach ca. 48 h bis 72 h erreicht.
Die Ausbeuten liegen bei 200 bis 300 mg/l.

d) Kultivierung von DSM 3816 im Fermenter

Ein Fermenter von 13 l Fassungsvermögen wird unter folgenden Bedingungen betrieben:
Bei 30° C Inkubationstemperatur und 300 UPM des Rührers werden 7 l Luft pro Minute in die Kulturflüssigkeit (Medium wie in Beispiel 1c) eingeleitet. Der Fermenter wird mit 500 ml der Vorkultur (siehe 1b) beimpft. Das Produktionsoptimum wird nach ca. 48 bis 76 h erreicht. Die Ausbeuten liegen bei ca. 300 bis 400 mg Amycin/l.

2. Aufarbeitung von 20 l Kulturbrühe

Die Kulturbrühe wird filtriert. Dad Mycel wird mit Essigester (3×500 ml) gewaschen und anschließend mit Methanol extrahiert. (3×500 ml). Der Alkohol wird im Vakuum abdestilliert und der erhaltene Rückstand (27 g) an zwei Kieselgelsäulen (500 g) mit Essigester/Methanol/Wasser (6:1:1; v:v:v) chromatographiert (Mitteldruck-Chromatographie). Man erhält 8,4 g Amycin.

Das Kulturfiltrat wird 2 × 20 l Essigester gewaschen und anschließend mit 3 × 20 l Chloroform/Methanol (1:1, v:v) extrahiert. Die vereinigten organischen Phasen werden im Vakuum zu einem Sirup eingeengt (78 g). Der Rückstand wird an Kieselgel mit Essigester/Methanol/Wasser 6:1:1 wie oben chromatographiert. Man erhält 5,3 g Amycin und 450 mg der entsprechenden Desmalonylverbindung, in der $R^1$, alle $R^2$, $R^3$ und $R^4$ Wasserstoff bedeuten.

Amycin:
MS (FAB)/$MH^+$ = 1229
$^{13}$C-NMR(75 MHZ) in $CD_3OD$: $\delta$ = 176,9; 174,2; 174,1; 171,7; 171,3; 156,6; 99,1 ppm
IR(KBr) ($cm^{-1}$) = 3400, 2860, 1720, 1640, 1600, 1460, 1385
Desmalonylverbindung:
MS (FAB): $MH^+$ = 1057
$^{13}$C-NMR(75 MHZ) in $CD_3OD$: $\delta$ = 177,1; 156,6; 99,1 ppm;

3. Herstellung der Desmalonyl-Verbindung ($R^1$, alle $R^2$, $R^3$, $R^4$ = H) und des in 22/23 Stellung dehydratisierten Derivats

1 g Amycin (0,81 mmol) werden in 75 ml Methanol mit 100 mg Natriumhydrid 24 Stunden bei Raumtemperatur gerührt. Nach der Neutralisation mit 5N Salzsäure wird der Alkohol im Vakuum abdestilliert. Der erhaltene Sirup wird an 100 g Kieselgel mit Essigester/Methanol/Wasser (15:2:1, 800 ml und 8:2:1; v:v:v.) chromatographiert. Die Kristallisation aus Methanol/Essigester ergibt 720 mg (72 %) der Desmalonylverbindung. Weiterhin werden 80 mg (8 %) der 22/23 dehydratisierten Verbindung (Formel IIa) erhalten.

22/23 dehydratisierte Verbindung: MS (FAB): $MH^+$ = 1039

4. Herstellung von $R^3$-Alkylamycin

1 g Amycin (0,81 mmol) werden mit einem Gemisch aus 1,5 ml Acetylchlorid und 75 ml Methanol 15 min bei
Raumtemperatur gerührt. Nach der Neutralisation mit 5N NaOH wird im Vakuum zu einem Sirup eingeengt. Chromatographie mit HPLC an RP-C-18 [30 min., Methanol/$H_2O$: (6:4; v:v) und dem Gradienten Methanol/Wasser von 6:4 auf 99:1 in 10 min. und 30 min. mit Methanol/Wasser 99:1] ergibt 760 mg (70 %) Methylamycin.

MS (FAB) : $MH^+$ = 1243
Die Anwendung dieser Vorschrift mit Ethanol, n-Butanol, Allylalkohol und Hexylalkohol führt zu den Verbindungen Ethyl-, Butyl-, Allyl-, und Hexylamycin.

5. Herstellung der $R^3$-Alkyl-desmalonylderivate ($R^1$, alle $R^2$ $R^4$ = H; $R^3$ = Alkyl) und der Diethylverbindung ($R^1$, alle $R^2$ = H, $R^3$, $R^4$ = Alkyl)

500 mg Desmalonylverbindung gemäß Beispiel 3 werden zu einer Mischung von 0,1 ml Acetylchlorid in 5 ml Ethanol gegeben und 20 min. bei Raumtemperatur gerührt. Die methanolische Lösung wird direkt an Kieselgel mit Chloroform/Methanol (8:2; v:v) chromatographiert. Es werden 400 mg (80%) $R^3$-Ethyl-desmalonylderivat und 70 mg (14 %) der Diethylverbindung erhalten.

$R^3$-Ethyl-desmalonylderivat: MS (FAB) $MH^+$ = 1085
Diethylverbindung: MS (FAB) $MH^+$ = 1113

Anwendung der analogen Vorschrift mit Propanol, n-Butanol, Allylalkohol und n-Hexylalkohol ergibt die entsprechenden Derivate.

6. Herstellung der Verbindung der Formel Ib

500 mg Desmalonylverbindung gemäß Beispiel 3 werden in 50 ml Methanol mit 500 mg Natriumborhydrid versetzt und 24 Stunden bei Raumtemperatur gerührt. Der Überschuß an Natriumborhydrid wird durch Zugabe von Eisessig zerstört. Abdestillieren des Lösungsmittels und Chromatographie an Dextran ($^{(R)}$Sephadex LH 20) mit Methanol als Elutionsmittel ergibt 450 mg (90 %) der Vebindung der Formel Ib, in der $R^1$ und alle $R^2$ Wasserstoff bedeutet

MS (FAB) $MH^+$ = 1159
Analoge Reduktion von Amycin ergibt 420 mg (84 %) der Verbindung der Formel Ib in der $R^1$ und ein $R^2$ Malonyl und der restlichen $R^2$ Wasserstoff bedeuten
MS (FAB) $MH^+$ = 1231

7. Verwendung von Amycin und der entsprechenden Desmalonylverbindung als fungizides Mittel

a) Filterpapierscheibchen von 6 mm Durchmesser werden mit je 20 µl Wirkstoff gemäß Beispiel 2 und 3 unterschiedlicher Konzentrationen (s. Tabelle 1 und 2) gleichmäßig benetzt und auf ein je nach Pilzart unterschiedlicher Agarmedium aufgelegt. Dem Agar werden zuvor in noch flüssigem Zustand je Petrischale (10 ml Agar, 90 mm Durchmesser) 0,5 ml Suspensionskultur des Testorganismus (ca. $10^5$ - $10^6$ Kondidien/l ml) zugegeben und die 10 behandelten Agarplatten anschließend bei 25 ° C bebrütet. Nach 3- bis 4-tägiger Inkubation wird die Inhibitionszone als Maß der Pilzhemmung gemessen und in mm angegeben. Eine Hemmzone von 10 mm wird als MIC-Wert (minimal inhibition concentration) des betreffenden Wirkstoffes in ppm Wirkstoff ausgedrückt.

Tabelle Nr. 1

Quantitativer Bioassay von Amycin nach Beispiel 2

Durchmesser der Bioaktivitätszone (mm)/20 µl Filterpapierscheibe

| Konzentration | Botrytis cinerea | | Cercospora | Piricularia | Alternaria | Fusarium | Penicillium |
|---|---|---|---|---|---|---|---|
| ppm (µl/ml) | sensitiv | resistent | beticola | oryzae | alternata | culmorum | digitatum |
| 2000 | 28 | 26 | 22 | 28 | 34 | 18 | 12 |
| 1000 | 24 | 24 | 20 | 26 | 28 | 16 | 10/12 * |
| 500 | 22 | 18 | 10 | 20 | 26 | 14 | 12 * |
| 250 | 16 | 12 | 12/16 * | 18 | 22 | 14/16 * | 10 * |
| 125 | 12 | 12 * | 16 * | 14 | 16 | 12 * | 0 |
| 60 | 10 | 10 * | 14 * | 10 | 14 | 10 * | 0 |
| 30 | 10 h | 0 | 12 * | 14 * | 12 * | 0 | 0 |
| 15 | 0 | 0 | 0 | 0 | 0 | 0 | 0 |

* = unvollständige Inhibierung, jedoch verkümmertes Mycel

### Tabelle Nr. 2

Quantitativer Bioassay der Desmalonylverbindung nach Beispiel 3
Durchmesser der Bioaktivitätszone (mm)/20 µl Filterpapierscheibe

| Konzentration ppm (µl/ml) | Botrytis cinerea sensitiv | Botrytis cinerea resistent | Cercospora beticola | Piricularia oryzae | Alternaria alternata | Fusarium culmorum | Penicillium digitatum |
|---|---|---|---|---|---|---|---|
| 2000 | 14 | 16 | 14 | 16 | 16 | 12 | 10 |
| 1000 | 12 | 14 | 12 | 14 | 14 | 10 | 8 |
| 500 | 12 | 12 | 10 | 12 | 12 | 10 | 8/10 * |
| 250 | 10/12 * | 10/12 * | 10 | 12 | 12 | 10 | 10 * |
| 125 | 12 * | 12 * | 10 * | 8 | 10 * | 0 | 0 |
| 60 | 10 * | 10 * | 0 * | 0 | 0 | 0 * | 0 |
| 30 | 0 | 0 | 0 | 0 | 0 | 0 | 0 |

b) Ackerbohnen (Sorten "Herz Freya" oder "Frank's Ackerperle") werden bei ca. 26 bis 28° C und 60 % relativer Luftfeuchte angezogen. 14 Tage nach der Aussaat (Wuchshöhe 13 bis 16 cm) sind die Pflanzen für die Versuche geeignet.
Nach Vorbereitung der Pflanzen für den Versuch erfolgt die Applikation der Versuchspräparate gemäß Beispiel 2 und 3 mit den in Tabelle 3 und 4 angegebenen Konzentrationen mit einem Glaszerstäuber bei 0,3 bis 0,5 bar Überdruck auf die Blätter der Ackerbohnen. Die behandelten Pflanzen werden zum

Trocknen abgestellt und ca. 3 Stunden später inokuliert.

Mit den frischen Konidien von Botrytis cinerea werden Sporensuspensionen mit $4 \times 10^5$ Sporen pro 1 ml hergestellt. Anschließend werden mit Hilfe eines fein sprühenden Glaszerstäubers die Sporensuspension gleichmäßig aus die Vicia faba Pflanzen appliziert. Die Pflanzen werden in einer Klimakammer bei 20 bis 22°C und ca. 99 % relativer Luftfeuchte aufgestellt. Die Infektion der Pflanzen äußert sich in der Bildung schwarzer Flecken auf Blättern und Stengeln und läßt die Pflanzen bei starkem Befall zusammenbrechen. Die Auswertung der Versuche erfolgt 3 bzw. 6 Tage nach der Inokulation.

Der Wirkungsgrad der Wirkstoffe wird ausgedrückt in % im Vergleich zur unbehandelten, infizierten Kontrolle.

**Tabelle 3:**

| Konzentration in ppm Wirkstoff | Wirkungsgrad [%] von Amycin gemäß Beispiel 2 gegenüber Botrytis cinerea bei ppm Wirkstoff BCM- und Iprodion-sensibler (s) und resistenter (r) Stamm | |
|---|---|---|
| | s | r |
| 1000 | 95 | 95 |
| 500 | 90 | 95 |
| 250 | 90 - (85) | 95 |
| 125 | 90 | 95 |
| 60 | 40 | 65 |
| Kontrolle | 0 | 0 |

**Tabelle 4:**

| Konzentration in ppm Wirkstoff | Wirkungsgrad [%] der Desmalonylverbindung nach Beispiel 3 gegenüber Botrytis cinerea bei ppm Wirkstoff BCM- und Iprodion-sensibler (s) und resistenter (r) Stamm | |
|---|---|---|
| | s | r |
| 1000 | 70 | 90 |
| 500 | 65 | 85 |
| 250 | 65 | 80 |
| 125 | 40 | 65 |
| 60 | 0 | 0 |
| Kontrolle | 0 | 0 |

c) Apfelunterlagen (EM IX) wurden im 4-Blattstadium mit den in der nachfolgenden Tabelle aufgeführten

Verbindungen in den Anwendungskonzentrationen von 500, 250, 125, 60 und 30 mg Wirkstoff/Liter Spritzbrühe gleichmäßig behandelt.

Nach Antrocknen des Wirkstoffbelages wurden Pflanzen mit Konidien des Apfelschorfs (Venturia inaqualis) stark inokuliert und tropfnaß in eine Klimakammer gestellt, deren Temperatur ca. 22°C und deren relative Luftfeuchtigkeit ca. 100 % betrug. Nach einer Infektionszeit von 48 Stunden kamen die Pflanzen in ein Gewächshaus mit ca. 18°C und einer relativen Luftfeuchtigkeit von 95 - 100 %.

Nach einer Inkubationszeit von 14 Tagen wurden die Pflanzen auf Befall mit Apfelschorf (Venturia inaequalis) untersucht. Die Beurteilung des Befalls erfolgte wie üblich nach Augenschein. Der Wirkungsgrad der Wirkstoffe bei Apfelschorf wird ausgedrückt in % im Vergleich zur unbehandelten, infizierten Kontrolle.

**Tabelle 7**

| Wirkstoff gemäß Beispiele 2 und 3 | Wirkungsgrad [%] bei Schorf (Venturia inaequalis) mit Wirkstoff [ppm] | | | | |
|---|---|---|---|---|---|
| | 500 | 250 | 125 | 60 | 30 |
| Amycin | 90 | 85 | 75 | 40 | 40 |
| Desmalonylderivat | 75 | 65 | 40 | 40 | 0 |
| unbehandelte infizierte Pflanzen | | | 0 | | |

8. Vergleich der fungiziden Wirkung von Amicin und der Desmalonylverbindung mit Niphimycin

Man verfährt gemäß Beispiel 7a setzt jedoch als Testsubstanz Niphimycin ein.
Die Ergebnisse sind in der folgenden Tabelle 8 aufgeführt.

Tabelle Nr. 8

Quantitativer Bioassay von Niphimycin

Durchmesser der Bioaktivitätszone (mm)/20 µl Filterpapierscheibe

| Konzentration ppm (µl/ml) | Botrytis cinerea sensitiv | Botrytis cinerea resistent | Cercospora beticola | Piricularia oryzae | Alternaria alternata | Fusarium culmorum | Penicillium digitatum |
|---|---|---|---|---|---|---|---|
| 2000 | 24 | 24 | 24 | 28 | 28 | 14 | 12 |
| 1000 | 20 | 18 | 18 | 22 | 20 | 10 | 10/12 * |
| 500 | 14 | 10 | 12 | 18 | 14 | 12 * | 12 * |
| 250 | 10 | 12 | 10 | 12 | 12 | 10 * | 10 * |
| 125 | 10 * | 0 | 0 | 10 * | 10 * | 0 | 0 |
| 60 | 0 | 0 | 0 | 0 | 0 | | 0 |

* = unvollständige Inhibierung, jedoch verkümmertes Mycel

## Patentansprüche

**1.** Die Verbindungen der Formel Ia,

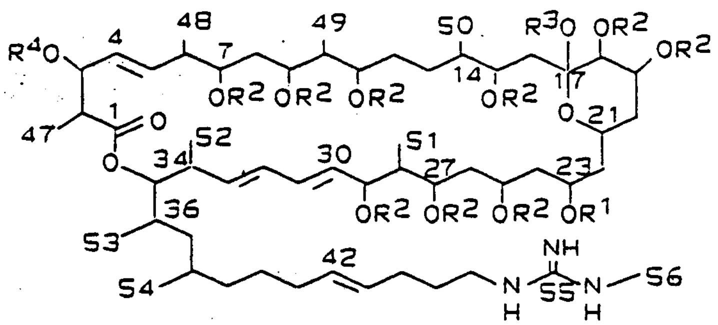

Ia

in der jeweils unabhängig voneinander

$R^1$ und $R^2$ Wasserstoff oder eine Malonylgruppe ist, wobei höchstens ein $R^2$-Substituent eine Malonylgruppe darstellt,

$R^3$ und $R^4$ Wasserstoff oder $C_1$-$C_6$ Alkyl oder Allyl bedeutet,

sowie die Verbindungen der Formel IIa, die in 22/23 Stellung ungesättigt sind,

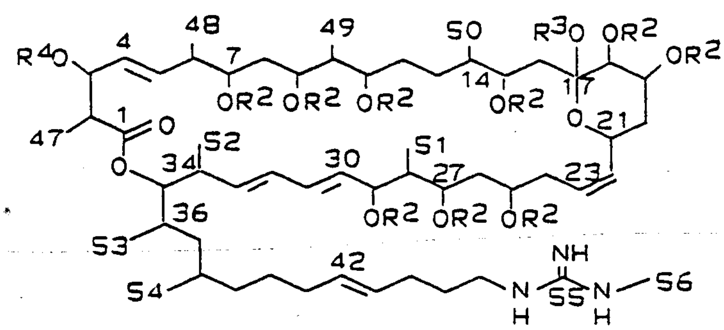

IIa

ausgenommen die Kombinationen in Verbindungen der Formel Ia, in denen

a) $R^1$ Malonyl und alle $R^2$, $R^3$, $R^4$ Wasserstoff,

b) $R^1$ Malonyl, alle $R^2$ und $R^4$ Wasserstoff und $R^3$ Methyl oder

c) $R^1$, alle $R^2$ und $R^4$ Wasserstoff und $R^3$ Methyl ist,

eingeschlossen weiterhin Verbindungen der Formel Ib,

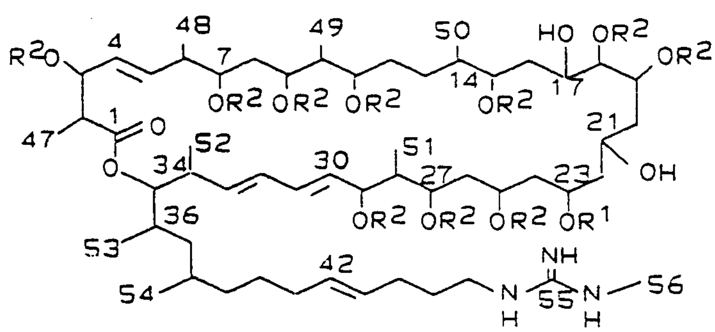

Ib

in der $R^1$ und $R^2$ die obengenannte Bedeutung haben wobei höchstens ein $R^2$-Substituent eine Malonylgruppe darstellt, sowie die Verbindungen der Formel IIb, die in 22/23 Stellung ungesättigt sind.

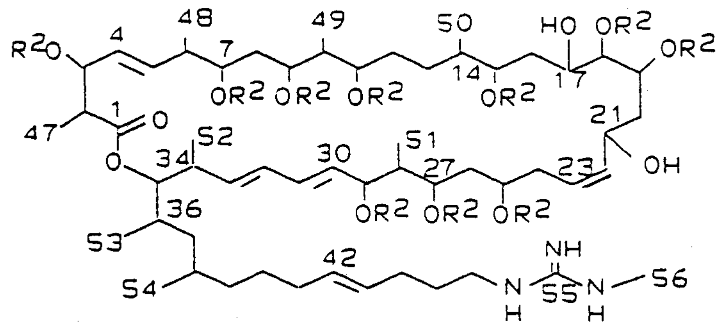

IIb

ausgenommen jene Verbindungen der Formel Ib, in welcher $R^1$ und alle $R^2$ Wasserstoff bedeuten.

**2.** Die Verbindung der allgemeinen Formel Ia nach Anspruch 1, in der $R^1$ und ein $R^2$-Rest Malonyl und $R^3$ und $R^4$ Wasserstoff bzw. $R^1$, alle $R^2$, $R^3$ und $R^4$ Wasserstoff bedeuten.

**3.** Verfahren zur Herstellung der Verbindung der allgemeinen Formel Ia, IIa und Ib und IIb, dadurch gekennzeichnet, daß
a) Streptomyces parvullus, DSM 3816, sowie seine Varianten und Mutanten in einem Nährmedium kultiviert werden bis sich die Verbindung der Formel Ia, in der $R^1$ und ein $R^2$-Rest Malonyl sowie $R^3$ und $R^4$ Wasserstoff bedeutet, sowie die Verbindung, in der $R^1$, alle $R^2$, $R^3$ und $R^4$ Wasserstoff bedeuten, in der Kultur anhäuft und diese gegebenenfalls
b) isoliert und derivatisiert wird.

**4.** Verfahren nach Anspruch 3, dadurch gekennzeichnet, daß DSM 3816 in einer Nährlösung fermentiert wird, die Sojamehl und Mannit enthält.

**5.** Verfahren nach Anspruch 3 oder 4, dadurch gekennzeichnet, daß bei einer Temperatur von 25° bis 30°C fermentiert wird.

**6.** Verfahren nach einem oder mehreren der Ansprüche 3 bis 5, dadurch gekennzeichnet, daß bei einem pH-Wert zwischen 6 und 8 fermentiert wird.

**7.** Verbindung der Formel Ia, IIa und Ib und IIb gemäß Anspruch 1, erhältlich nach einem oder mehreren der Ansprüche 3 bis 6.

**8.** Verwendung der Verbindung der Formel Ia, IIa und Ib und IIb gemäß Anspruch 1 zur Herstellung von Heilmitteln.

**9.** Verwendung der Verbindung der Formel Ia, IIa und Ib und IIb gemäß Anspruch 1 zur Herstellung von Fungiziden.

**10.** Streptomyces parvullus, DSM 3816, sowie seine Varianten und Mutanten, sofern sie die Verbindung der Formel Ia nach Anspruch 2, in der $R^1$ und ein $R^2$-Rest Malonyl sowie $R^3$ und $R^4$ Wasserstoff bedeutet und/oder die Verbindung, in der $R^1$, alle $R^2$, $R^3$ und $R^4$ Wasserstoff bedeutet, synthetisieren.

## Claims

**1.** The compounds of the formula Ia,

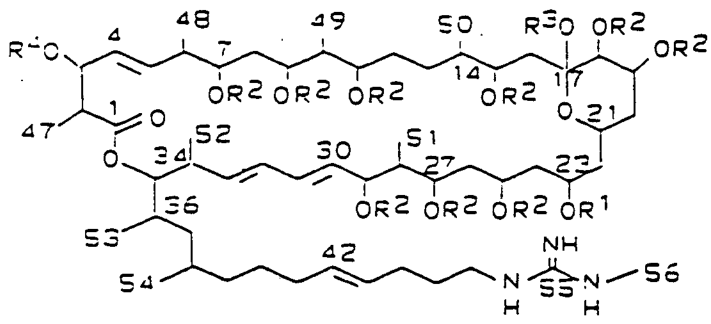

Ia

in which, in each case independently of one another, $R^1$ and $R^2$ are hydrogen or a malonyl group, not more than one $R^2$ substituent representing a malonyl group, $R^3$ and $R^4$ denote hydrogen or $C_1$-$C_6$ alkyl or allyl, and the compounds of the formula IIa which are unsaturated in the 22/23 position,

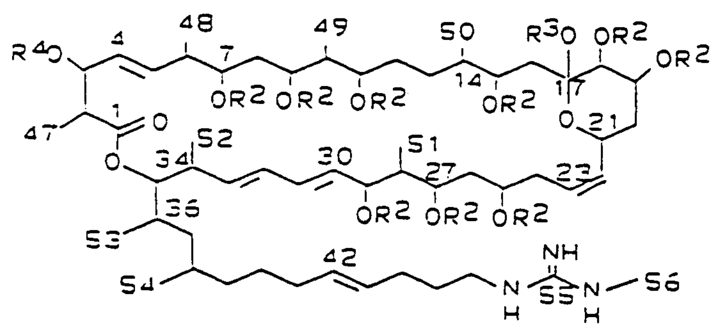

IIa

excepting the combinations in compounds of the formula Ia in which

a) $R^1$ is malonyl and all $R^2$, $R^3$, and $R^4$ are hydrogen,

b) $R^1$ is malonyl, all $R^2$ and $R^4$ are hydrogen, and $R^3$ is methyl, or

c) $R^1$, all $R^2$ and $R^4$ are hydrogen, and $R^3$ is methyl,

including, furthermore, compounds of the formula Ib,

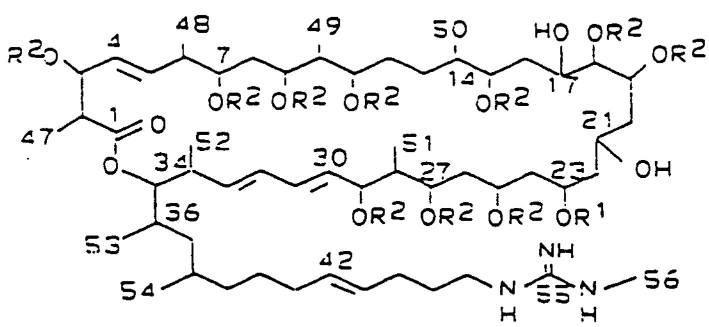

Ib

in which $R^1$ and $R^2$ have the abovementioned meaning, not more than one $R^2$ substituent representing a malonyl group, as well as the compounds of the formula IIb which are unsaturated in the 22/23 position.

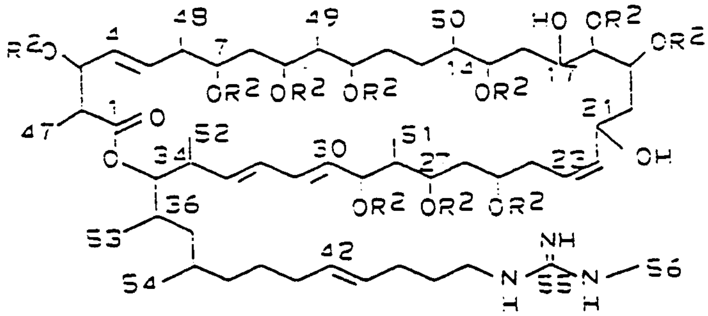

IIb

excepting the compound of the formula Ib in which $R^1$ and all $R^2$ denote hydrogen.

**2.** The compound of the formula Ia as claimed in claim 1, in which $R^1$ and one $R^2$ radical denote malonyl, and $R^3$ and $R^4$ denote hydrogen, or $R^1$, all $R^2$, $R^3$ and $R^4$ denote hydrogen.

**3.** A process for the preparation of the compound of the formula Ia, IIa or Ib or IIb, which comprises
a) cultivation of Streptomyces parvullus, DSM 3816, and its variants and mutants, in a nutrient medium until the compound of the formula Ia in which $R^1$ and one $R^2$ radical denote malonyl, and $R^3$ and $R^4$ denote hydrogen, and the compound in which $R^1$, all $R^2$, $R^3$ and $R^4$ denote hydrogen, accumulate in the culture, and where appropriate
b) isolation and derivatization of the latter.

**4.** The process as claimed in claim 3, wherein DSM 3816 is fermented in a nutrient solution which contains soybean meal and mannitol.

**5.** The process as claimed in claim 3 or 4, wherein fermentation is carried out at a temperature of 25° to 30°C.

**6.** The process as claimed in one or more of claims 3 to 5, wherein fermentation is carried out at a pH between 6 and 8.

**7.** A compound of the formula Ia, IIa or Ib or IIb as claimed in claim 1, obtainable as claimed in one or more of claims 3 to 6.

**8.** The use of the compound of the formula Ia, IIa or Ib or IIb as claimed in claim 1 for the preparation of medicines.

**9.** The use of the compound of the formula Ia, IIa or Ib or IIb as claimed in claim 1 for the preparation of fungicides.

**10.** Streptomyces parvullus, DSM 3816, and its variants and mutants as long as they synthesize the compound of the formula Ia as claimed in claim 2, in which $R^1$ and one $R^2$ radical denote malonyl, and $R^3$ and $R^4$ denote hydrogen, and/or the compound in which $R^1$, all $R^2$, $R^3$ and $R^4$ denote hydrogen.

## Revendications

**1.** Composés de formule Ia

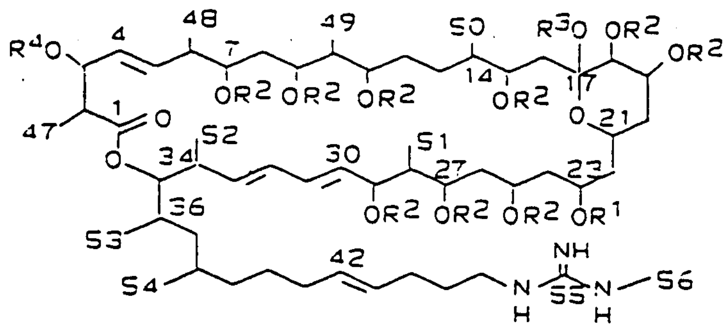

Ia

dans laquelle, indépendamment les uns des autres,

$R^1$ et $R^2$ sont un atome d'hydrogène ou le groupe malonyle, au maximum un substituant $R^2$ représentant le groupe malonyle,

$R^3$ et $R^4$ représentent un atome d'hydrogène ou un groupe alkyle en $C_1$-$C_6$ ou allyle,

et composés de formule IIa qui sont insaturés en position 22/23

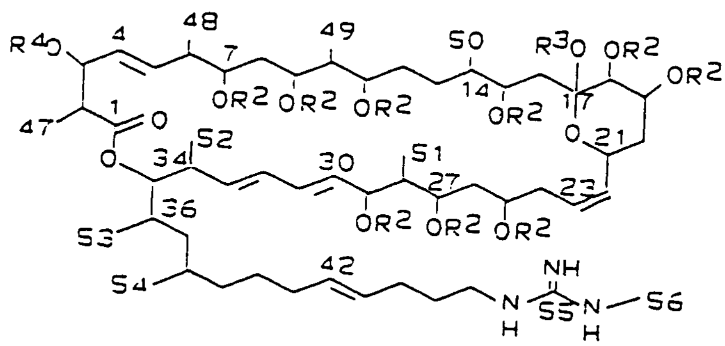

IIa

à l'exception des combinaisons, dans des composés de formule Ia, dans lesquelles

a) $R^1$ est le groupe malonyle et tous les radicaux $R^2$, $R^3$, $R^4$ sont des atomes d'hydrogène,

b) $R^1$ est le groupe malonyle, tous les radicaux $R^2$ et $R^4$ sont des atomes d'hydrogène et $R^3$ est le groupe méthyle, ou

c) $R^1$, tous les radicaux $R^2$ et $R^4$ sont des atomes d'hydrogène et $R^3$ est le groupe méthyle,

y compris en outre les composés de formule Ib

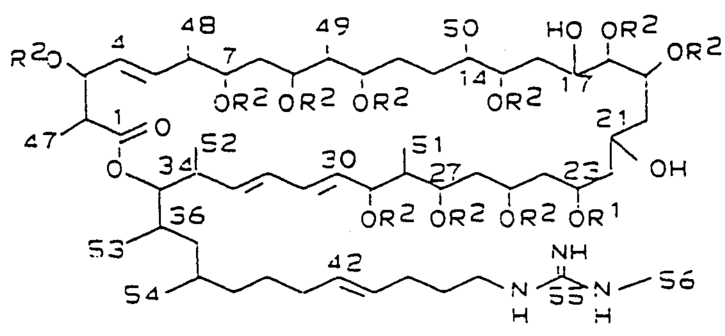

Ib

dans laquelle $R^1$ et $R^2$ ont les significations données plus haut, au maximum un substituant $R^2$ représentant le groupe malonyle, ainsi que les composés de formule IIb qui sont insaturés en position 22/23,

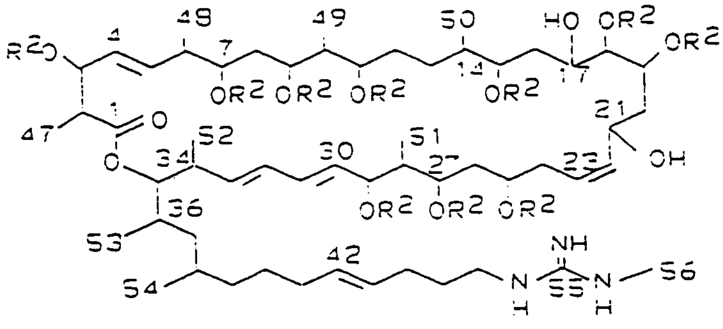

IIb

à l'exception des composés de formule Ib dans lesquels $R^1$ et tous les radicaux $R^2$ représentent des atomes d'hydrogène.

**2.** Composés de formule générale Ia selon la revendication 1, dans laquelle $R^1$ et un radical $R^2$ représentent le groupe malonyle et $R^3$ et $R^4$ représentent des atomes d'hydrogène, ou $R^1$, tous les radicaux $R^2$, $R^3$ et $R^4$ représentent des atomes d'hydrogène.

**3.** Procédé pour la préparation du composé de formules générales Ia, IIa et Ib et IIb, caractérisé en ce que

a) on cultive dans un milieu nutritif Streptomyces parvullus DSM 3816, ainsi que ses mutants et variants, jusqu'à ce que s'accumulent dans la culture le composé de formule Ia dans lequel $R^1$ et un radical $R^2$ représentent le radical malonyle et $R^3$ et $R^4$ représentent des atomes d'hydrogène, ainsi que le composé dans lequel $R^1$, tous les radicaux $R^2$, $R^3$ et $R^4$ représentent des atomes d'hydrogène, et éventuellement

b) on les isole et les transforme en dérivés.

**4.** Procédé selon la revendication 3, caractérisé en ce que l'on cultive DSM 3816 par fermentation dans un milieu de culture qui contient de la farine de soja et du mannitol.

**5.** Procédé selon la revendication 3 ou 4, caractérisé en ce que l'on effectue la fermentation à une température de 25 à 30 ° C.

**6.** Procédé selon une ou plusieurs des revendications 3 à 5, caractérisé en ce que l'on effectue la fermentation à un pH compris entre 6 et 8.

**7.** Composé de formules Ia, IIa et IIa et IIb selon la revendication 1, pouvant être obtenu selon une ou plusieurs des revendications 3 à 6.

**8.** Utilisation du composé de formules Ia, IIa et Ib et IIb selon la revendication 1, pour la fabrication de médicaments.

**9.** Utilisation du composé de formules Ia, IIa et Ib et IIb selon la revendication 1, pour la fabrication de fongicides.

**10.** Streptomyces parvullus DSM 3816, ainsi que ses variants et mutants, dans la mesure où ils synthétisent le composé de formule Ia selon la revendication 2, dans lequel $R^1$ et un radical $R^2$ représentent le groupe malonyle et $R^3$ et $R^4$ représentent des atomes d'hydrogène et/ou le composé dans lequel $R^1$, tous les radicaux $R^2$, $R^3$ et $R^4$ représentent des atomes d'hydrogène.